# EUROPEAN PATENT SPECIFICATION

(11) **EP 1 611 871 B1**
(45) Date of publication and mention of the grant of the patent: **20.02.2008**
(21) Application number: 04254018.7
(22) Date of filing: 02.07.2004
(51) Int. Cl.: A61F 2/38

(54) **Multiple piece modular patellar prosthetic system**
Modulare Patella-Implant Gelenkprothese mit unterschiedlichen Komponenten
Implant prosthétique patellaire avec multiples pièces

(43) Date of publication of application: 04.01.2006
(73) Proprietor: Zimmer Technology, Inc., Warsaw IN 46580 (US)
(72) Inventor: Burkinshaw, Brian, Pflugerville, TX 78660 (US); Brown, Steven, Pflugerville, TX 78660 (US)
(74) Representative: Mays, Julie

(56) References cited:
- EP-A- 0 353 921
- US-A- 4 240 162
- US-A- 5 702 465
- US-A1- 2002 128 719
- US-A1- 2002 173 852

## Description

### Field of the Invention

The present invention relates to a modular knee prosthetic system used to replace the natural knee and, more particularly, to a multi-piece modular patellar prosthetic system having various baseplates and articulation components that are interchangeable with each other.

### Background of the Invention

In the United States alone, over 200,000 knee replacements are performed each year. Degenerative arthritis, or the gradual degeneration of the knee joint, is the most common reason for these replacements. In this form or arthritis, cartilage and synovium surrounding the knee wear down so underlying bones grind directly on each other.

In knee arthroplasty, portions of the natural knee joint are replaced with prosthetic components. These components include a tibial component, a femoral component, and a patellar component. The femoral component generally includes a pair of spaced condyles that articulate with the tibial component. These condyles form a trochlear groove in which the articulating surface of the patellar component moves. The components are made of materials that exhibit a low coefficient of friction when they articulate against one another.

When the articulating ends of both the femur and tibia are replaced, the procedure is referred to as total knee replacement or TKR. Much effort has been devoted to performing TKR that restores normal, pain-free functions of the knee for the lifetime of the prosthetic components.

Unfortunately, patients can experience problems with the prosthetic knee after a total knee replacement surgery. If a problem occurs, a patient may need a revision surgery wherein some or all of the prosthetic components are replaced. Historically, problems associated with the patellar prosthesis are responsible for as many as 50% of all knee implant revisions. More particularly, complications with the patello-femoral joint or patello-femoral dysfunction are the primary cause of failure in TKR.

One option in a TKR or revision surgery is to implant a prosthetic patellar component. The patellar component has a metallic back or baseplate that is permanently fixed to the patellar bone. Metal baseplates were introduced to provide a more even stress distribution on the natural patella and provide the option for either cement or cementless fixation. An articulation or bearing component is permanently connected to the baseplate to form the prosthetic patellar component. The articulation component is formed from metal or a polymer, such as ultra-high molecular weight polyethylene (UHMWPE).

Despite current advances in the design of prosthetic knees, the patellar component still fails and must be replaced in a revision surgery. Failure of the patellar component occurs for a multitude of reasons. In some instances, the articulation component becomes loose or worn through repeated use. Obviously then, this component must be replaced.

A modular patellar prosthetic system according to the preamble of claim 1 is disclosed in US 2002/128719.

As one disadvantage with current patellar components, replacement of the articulation or bearing component during a revision surgery can be impractical, difficult, or unhealthy for the natural patella. After the initial TKR surgery, the baseplate becomes firmly fixed to the host patellar bone. In present patellar prosthetic designs, the articulation component is permanently attached to the baseplate. So, removal of the articulation component alone is not an option. Instead, both the baseplate and the articulation component must be removed and then replaced. Removing the baseplate from the natural patellar bone is undesirable since healthy bone stock can be damaged or removed from the patella. Further, the stress associated with removing the baseplate during a revision surgery can fracture the natural patella. The patellar bone stock may already be thin or weak, and forcing or prying the baseplate from the bone can damage the patella.

Since removing the baseplate from the patella can have serious, unwanted consequences, surgeons have few options. Manufacturers do not provide modular articulation components that are designed to be removed from the baseplate during a revision surgery. In the past, some attempts have been made to forceably remove or pry apart the articulation component from the baseplate during a revision surgery. Manufacturers, however, would not recommend such a procedure if the components were not designed for this use.

It, therefore, would be advantageous to provide an implantable modular patellar prosthetic system having various baseplates and articulation components that are interchangeable with each other.

### Summary of the Invention

The present invention is directed toward a modular patellar prosthetic system used to replace a portion of the natural knee and, more particularly, to a multi-piece modular patellar prosthetic system having various baseplates and articulation components that are interchangeable with each other.

Each baseplate has a fixation surface and a bearing surface. The fixation surface is adapted to engage patellar bone and includes a plurality of pegs that extend outwardly from the surface to penetrate bone.

Each articulation component has an articulation surface and a bearing surface. The articulation surface has a smooth contour that is adapted to articulate with the femur or femoral prosthesis at the patello-femoral joint. This surface may have various shapes known to those skilled in the art, such as a hyperbolic paraboloid or dome-like configuration. The bearing surface of the articulation component is adapted to engage, either directly or indirectly, the bearing surface of the baseplate. In some embodiments, these surfaces are configured to slideably contact or articulate with each other. In other embodiments, the articulation component and baseplate anti-rotationally lock together.

An attachment mechanism couples the baseplate to the articulation component so the bearing surfaces are adjacent each other. The attachment mechanism is a separate component from the articulation component and baseplate and can have a variety of configurations to enable the articulation component to engage and disengage from the baseplate. In one embodiment, the attachment mechanism has a disc shape with a locking mechanism; and in other embodiments, the attachment mechanism has a ring shape. The attachment mechanism serves an important function as it enables the articulation component to attach and detach from the baseplate and provides a modular interface between the articulation component and various baseplates.

As one important advantage of the present invention, the articulation component is removeably connectable to the baseplate. In other words, even after the baseplate becomes permanently connected to the patellar bone, an articulation component can be readily attached or detached from the baseplate. During a revision surgery then, healthy bone stock of the natural patella will not be damaged or removed since the baseplate can be left attached to the patella.

As another advantage, an articulation component can be relatively easily removed from or attached to the baseplate. As such, nominal stress is placed on the natural patella as an old articulation component is removed and a new one is attached. The natural patella is thus less likely to fracture or otherwise become damaged during replacement of the articulation component.

As yet another advantage of the invention, multiple articulation components can be easily attached to an implanted baseplate. During a revision surgery then, the implanted articulation component can be removed from the baseplate and replaced with a new, sterile one. Further, multiple articulation components having various sizes and shapes can be attached to the baseplate. As such, the surgeon can choose from a variety of articulation components to meet the specific needs of the patient.

As yet another advantage, multiple articulation components can connect to multiple baseplates. The articulation components and baseplates can have different sizes and shapes and can interchange and connect to each other. The interchangeability between the various components gives the surgeon a wide array of options in selecting various articulation components and baseplates to meet the needs of the patient.

As yet a further advantage, the attachment mechanism is a separate component from the articulation component and baseplate. This mechanism enables the articulation component to be easily and repeatedly attached and detached from the baseplate.

Other objects and advantages of the present invention will be apparent from the following descriptions of a preferred embodiment with reference to the drawings.

### Brief Description of the Drawings

FIG. 1 is a top perspective view of a modular knee prosthetic system according to the invention that includes multiple baseplates removeably connectable with three different articulation components.
FIG. 2 is a bottom perspective view of the modular knee prosthetic system of FIG. 1.
FIG. 3 is a side view of one baseplate embedded in patellar bone with the three articulation components of FIG. 1 superimposed on the baseplate to illustrate the different sizes of articulation components.
FIG. 4 is an exploded top perspective view of an alternate embodiment of a modular knee prosthesis useable with the modular knee prosthetic system of the present invention.
FIG. 5 is an exploded bottom perspective view of the modular knee prosthesis of FIG. 4.
FIG. 6 is an exploded top perspective view of another alternate embodiment of a modular knee prosthesis useable with the modular knee prosthetic system of the present invention.
FIG. 7 is an exploded bottom perspective view of the modular knee prosthesis of FIG. 6.
FIG. 8 is a side perspective view of an assembled modular knee prosthesis of FIGS. 6 and 7.
FIG. 9 is another side perspective view of the assembled modular knee prosthesis of FIG. 8.
FIG. 10 is an exploded top perspective view of another alternate embodiment of a modular knee prosthesis useable with the modular knee prosthetic system of the present invention.
FIG. 11 is an exploded bottom perspective view of the modular knee prosthesis of FIG. 10.
FIG. 12 is a top perspective view of another modular knee prosthetic system that includes a baseplate removeably connectable with three different articulation components.
FIG. 13 is a bottom perspective view of the modular knee prosthetic system of FIG. 12.
FIG. 14 is a top perspective view of the modular knee prosthetic system of FIGS. 12 and 13 with a baseplate that is removeably connectable with five different articulation components.

### Detailed Description of the Preferred Embodiment

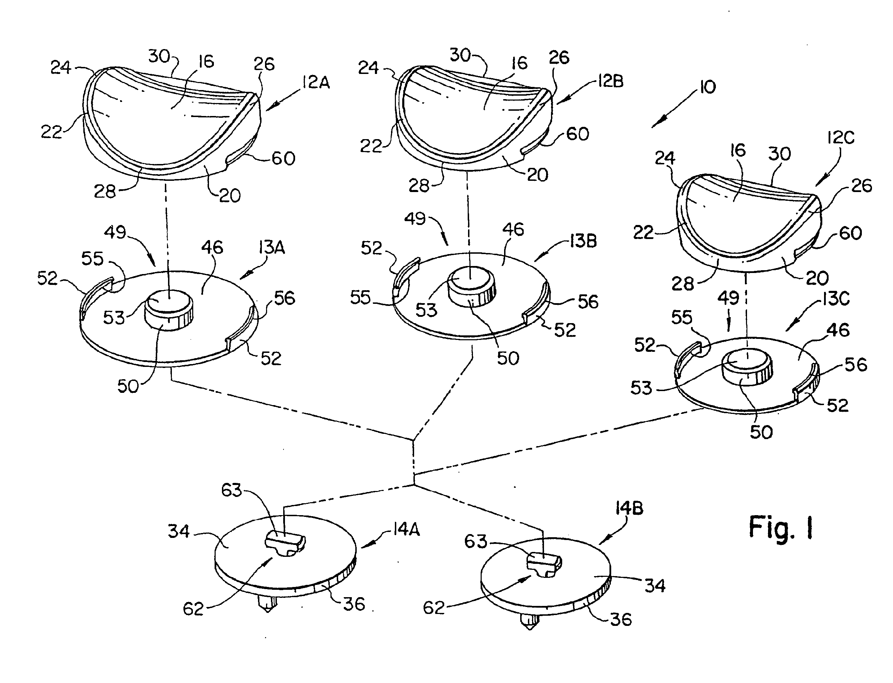

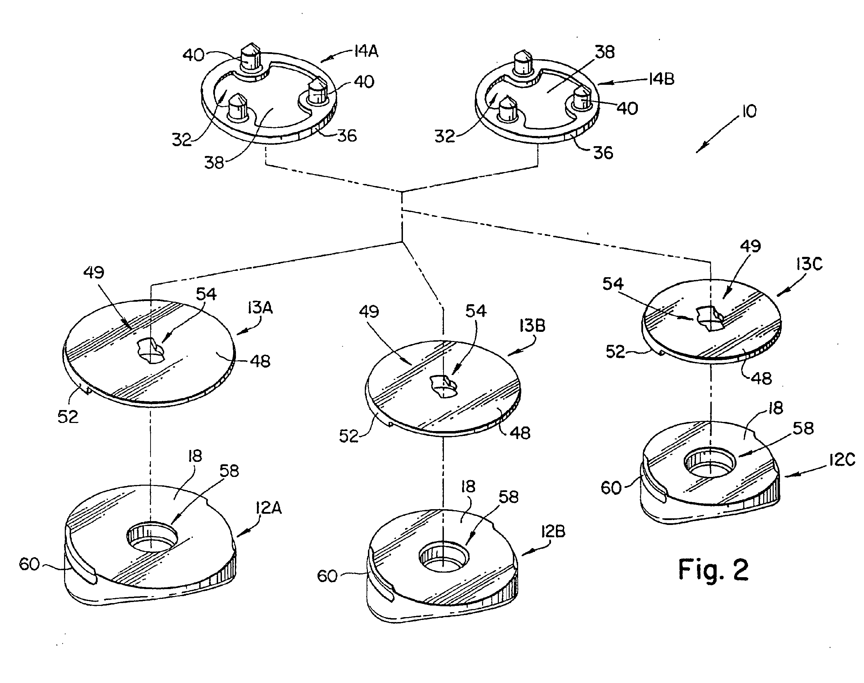

FIGS. 1-3 show a modular knee prosthetic system or kit 10 having a plurality of individual, implantable patellar prostheses. Prostheses of different sizes are shown wherein each prosthesis includes an articulation or bearing component 12A-12C, an attachment mechanism 13A-13C, and a base component or baseplate 14A and 14B.

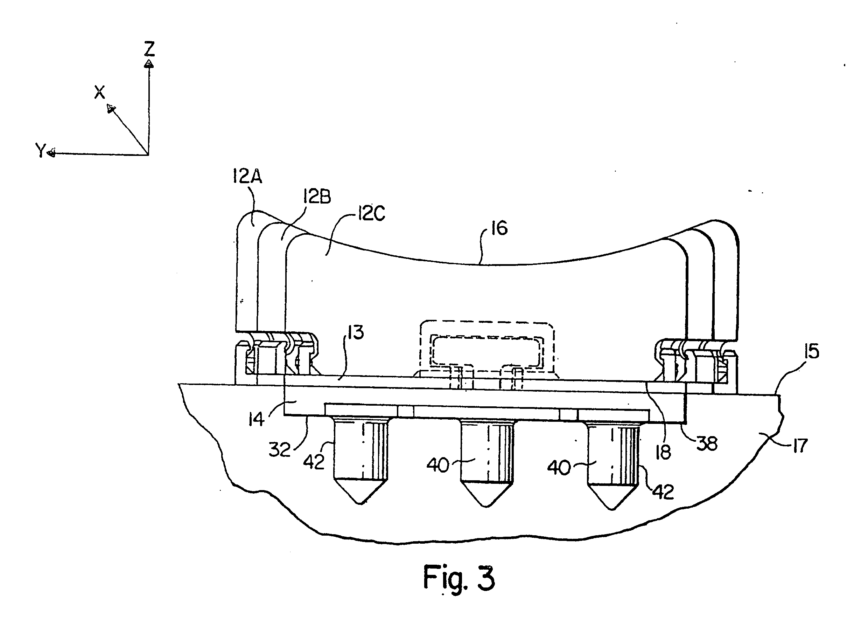

The articulation components and baseplates are shown relative to mutually orthogonal reference axes X, Y and Z (FIG. 3). When a prosthesis is implanted, reference axes X, Y and Z correspond, generally, to well known and accepted anatomical directional terms. The X axis extends generally in the medial-lateral direction, the Y axis extends generally in the inferior-superior direction, and the Z axis extends generally in the posterior-anterior direction. If the prosthesis were implanted on the left patella of a human patient, the ends of each of the X, Y, and Z axes marked with an arrowhead would point generally in the medial, superior, and posterior directions, respectively.

The present invention may be utilized in various knee surgeries known to those skilled in the art. As an example, during a TKR surgery, the patella is resected in a plane generally perpendicular to the anterior-posterior direction to remove a posterior portion of the patellar bone, leaving a resected planar bony surface 15 (FIG. 3). When a prosthesis is implanted, the Z axis lies perpendicular to the resected planar bony surface 15 of a patella 17, and the X and Y axes lie parallel to the resected planar bony surface.

The articulation components of the present invention are constructed of a biocompatible material having desirable wear and bearing friction properties, such as biocompatible metals and ultra-high molecular weight polyethylene (UHMWP). Examples of a suitable materials are Metasul® and Durasul® articulation components manufactured by Centerpulse Orthopedics Inc. of Austin, Texas.

Articulation component 12 includes two primary surfaces: An articulation surface 16 and a planar bearing surface 18 oppositely disposed from the articulation surface. The bearing surface 18 is generally perpendicular to the Z axis and spaced from the articulation surface 16 to define a thickness. A wall 20 extends around the outer perimeter of the articulation component and generally has an elliptical or round shape.

Articulation surface 16, in the preferred embodiment shown, is a hyperbolic paraboloid, also known as a "saddle" shape, in which the intersection of the surface 16 and wall 20 defines an undulating edge 22. Points 24 and 26 are at opposite ends of the "saddle" and designate the locations at which undulating edge 22 is at its maximum spacing from planar bearing surface 18. Points 24 and 26 are on the minor axis of wall 20, and are disposed relative to each other generally in the inferior-superior direction along the Y axis. Points 28 and 30 are at opposite sides of the "saddle" and designate the locations at which undulating edge 22 is at its minimum spacing from planar bearing surface 18. Points 28 and 30 are on the major axis of wall 20, and are disposed relative to each other generally in the medial-lateral direction, along the X axis. Articulation surface 16, so configured, ideally provides congruent sliding contact over an extensive range of articulation between articulation component 12 and the patellar articulation surface of a femoral prosthesis component (not shown) at the patello-femoral joint. Undulating edge 22 at points 24 and 26 at the high ends of the "saddle" functionally defines a ridge that can track the intercondylar groove of the femoral component during flexion and extension of the knee joint.

The baseplates of the present invention are constructed of a biocompatible material having desirable wear, bearing friction, and bone engaging properties that are known to those skilled in the art. Examples of such a material are UHMWPE, titanium, titanium alloys, zirconia ceramics, aluminum oxide ceramics, and cobalt chromium alloys.

Baseplate 14 includes a fixation surface 32 for engaging patellar bone 17, a planar bearing surface 34 generally perpendicular to the Z axis and spaced from the fixation surface 32, and an outer wall 36 that extends around the perimeter and is generally parallel to the Z axis. The baseplate generally has an elliptical or round shape to match the size and shape of the articulation component 12.

Fixation surface 32 includes a generally planar surface portion 38 adapted to engage resected planar bony surface 15 generally parallel thereto. The surface portion 38 can be adapted to directly engage and integrate with the patellar bone with or without bone cement. Planar surface portion 38, for example, can include surface texturing (such as grit-blasting or other roughened, textured surface) to promote osseointegration of baseplate 14. A coating of hydroxyapatite, ceramic, or porous metal are examples of surface texturing known to those skilled in the art. Such coatings can be applied with plasma spraying or sintering techniques. Suitable metals for sintering include titanium and its alloys and cobalt chromium alloys. Other materials and methods for providing a surface that favors osseointegration are well known in the art.

Fixation surface 32 also includes a plurality of pins or pegs 40 that extend downward from the surface. These pegs are evenly and symmetrically spaced apart and are integrally connected to fixation surface 32. The pegs 40 are sized and shaped to be received in correspondingly shaped bores 42 in patella 17 (FIG. 3). Specifically, each peg has a cylindrical body portion with a tapered or conical distal end. One skilled in the art will appreciate that the pegs can have various configurations and textures, such as a straight, ribbed, or tapered shape with a macro-textured surface to enhance fixation with bone cement or osseointegration.

One important advantage of the present invention is that the articulation component 12 is removeably connectable to the baseplate 14. Even after the baseplate becomes permanently connected to the patellar bone, an articulation component can be readily or easily attached and detached from the baseplate. The removeable or detachable connection between the baseplate and articulation component provides a modular knee prosthesis. As shown in FIGS. 1-3, a plurality of articulation components 12A-12C can connect to a plurality of baseplates 14A-14B. Each of the three articulation components has a similar shape with a different size. Three different sizes are shown, such as large, medium, and small sizes. Likewise, each of the baseplates has a similar shape with a different size. Two different sizes are shown, such as large and small. Together, the plurality of baseplates and plurality of articulation components form a modular knee prosthetic system.

FIG. 3 also illustrates how each articulation component would fit on one of the baseplates. It is important to note that any one of three different articulation components 12A-12C are engageable with and removable from any one of the baseplates 14A-14B. One skilled in the art will appreciate that the number of sizes can increase or decrease to offer a more diversified modular prosthetic knee system. Further, a variety of different shapes for both the articulation components and baseplates can be offered to provide a diversified modular knee prosthetic system.

During a TKR or other knee surgery, the surgeon can select any one of various sized and shaped articulation components to connect to any one of various sized and shaped baseplates. During a revision surgery for example, the implanted articulation component may be damaged, worn, or otherwise need replaced. The articulation component can be easily removed from the baseplate and replaced with a new, sterile one. At the same time though, the baseplate can be left undisturbed and attached to the patellar bone. Thus, a new and different articulation component can be engaged and connected intra-operatively to an existing baseplate previously implanted in the patient.

The coupling or attachment mechanism 13 enables the articulation component 12 and baseplate 14 to be connectable to and removeable from each other. Specifically, in the preferred embodiment, the attachment mechanism 13 has a flat, thin disc-shape with a first locking surface or side 46 adapted to engage the bearing surface 18 of the articulation component 12 and a second locking surface or side 48 adapted to engage the bearing surface 34 of the baseplate 14. The attachment mechanism 13 is provided as a completely separate component from both the articulation component 12 and baseplate 14 and has a locking mechanism 49 that enables the attachment mechanism to permanently connect to the articulation component and removeably connect to the baseplate.

The locking mechanism 49 includes a hub or pin 50 located in the center of the disk and two wings or shoulders 52 located on the periphery of the disk. On the first locking surface 46, the hub 50 projects outwardly and has cylindrical or tapered conical shape with a flat top surface 53. Hub 50 is hollow and includes a keyway or locking recess 54 projecting inwardly from the second locking surface 48. This keyway generally has an elongated rectangular shape and provides access to the enlarged hollow section inside the hub.

Each wing 52 extends upwardly from the first locking surface 46 and has an elongated, thin, rectangular shape defined by an inside wall 55 and an outside wall 56. The wings extend around the outer perimeter of the first locking surface 46 and thus have a curved shape.

The bearing surface 18 of the articulation component 12 has a centrally located bore or recess 58. This recess is sized and shaped to receive the hub 50 on locking mechanism 49. Articulation component 12 also includes a pair of cutouts or recesses 60 along the outer perimeter or wall 20. These cutouts are sized and shaped to receive the wings 52 of locking mechanism 49.

The bearing surface 34 of the baseplate 14 has a centrally located and outwardly extending pin 62. This pin has an elongated rectangular head portion 63 that is sized and shaped to extend into and through the keyway 54 of the second locking surface 48 of locking mechanism 49.

In operation, articulation component 12 and baseplate 14 are configured to engage each other in a removable locking or snap-retaining relationship. Specifically, the locking mechanism 49 is shaped and sized to connect to the articulation component 12. As the first locking surface 46 of the locking mechanism 49 is pressed or abutted against the bearing surface 18 of the articulation component 12, hub 50 projects into and engages with recess 58. At the same time, wings 52 project into and engage with cutouts 60. The wings 52 can be configured to be resilient and slightly deform outwardly to engage cutouts 60.

The connection between the articulation component 12 and the attachment mechanism 13 can be designed to be either permanent (i.e., not removable) or removable. Once the two components are connected, wings 52 are locked into cutouts 60 and prevent the attachment mechanism and articulation component from rotating relative to each other.

An important advantage of the present invention is that the articulation component 12 can repeatedly attach and detach from the baseplate 14. In this operation, the second locking surface 48 of attachment mechanism 13 is shaped and sized to removeably connect to and lock with the bearing surface 34 of the baseplate 14. As these two surfaces are pressed or abutted against each other, the head portion 63 of pin 62 extends through keyway 54 and into the hollow portion of hub 50. The articulation component 12 and accompanying attachment mechanism 13 can then be rotated 90° in either a clockwise or counterclockwise direction to secure and lock the baseplate 14 to the articulation component 12.

In order to remove the articulation component 12 from the baseplate 14, articulation component 12 and accompanying attachment mechanism 13 can then be rotated 90° in either a clockwise or counterclockwise direction to unlock the components.

FIGS. 1-3 show an attachment mechanism 13 formed as a disk with a locking mechanism adapted to engage both the articulation component and baseplate. One skilled in the art will appreciate that attachment mechanism can be altered without departing from the scope of the invention. As an example, the components of the locking mechanism can be switched, moved, and altered. Other embodiments as well are within the scope of the invention, and some of these embodiments are shown in the subsequent figures.

Another advantage of the present invention is that the articulation component, baseplate, and attachment mechanism are each formed as single, unitary pieces that are connectable together. The attachment mechanism enables the articulation component to removeably connect to the baseplate.

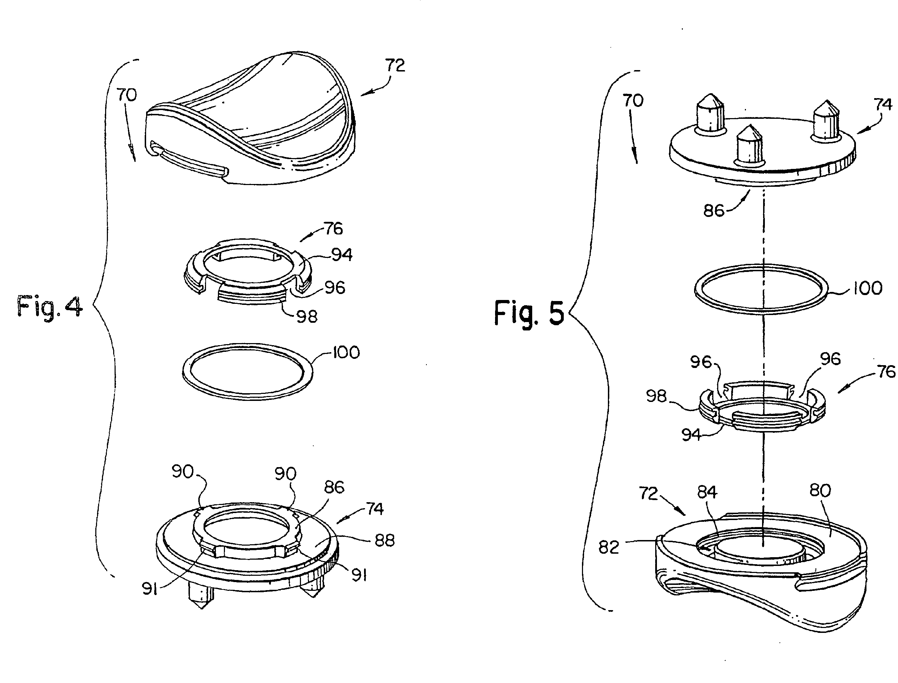

FIGS. 4 and 5 show an alternate modular knee prosthesis 70 that can be used with the various embodiments of the present invention. The prosthesis includes an articulation component 72, a baseplate 74, and an attachment mechanism 76. The articulation component and baseplate generally have a configuration similar to the articulation component 12 and baseplate 14 shown and described in connection with FIGS. 1-3. The primary differences between these embodiments centers around the attachment mechanism 76 and how it connects the articulation component to the baseplate.

Articulation component 72 has a bearing surface 80 with a circular channel or groove 82 that includes a recess 84 extending around the inner wall. The baseplate 74 includes a circular or annular protrusion 86 that extends outwardly from the bearing surface 88. The protrusion 86 has a rectangular cross-section with four rectangular legs 90 extending outwardly from the annular body. Each leg includes a lip, shoulder, or tang 91. The protrusion 86 is shaped and adapted to be received in the channel 82 of the articulation component 72.

The attachment mechanism 76 includes an annular or ring-shape body 94 with a locking mechanism formed as four rectangular cutouts 96 and a recess 98. The recess extends around an outer perimeter or surface of the body and is sized and shaped to receive a locking ring 100.

In operation, articulation component 72 and baseplate 74 are configured to engage each other in a locking relationship such that the two components can be connected and removed from each other. The attachment mechanism 76 is sized and shaped to fit into the circular recess 84 of the articulation component 72. Locking ring 100 fits in both recess 98 and recess 84 to connect and lock the attachment mechanism 76 to the articulation component 72.

As the bearing surface 80 of the articulation component is pressed or abutted against the bearing surface 88 of the baseplate 74, the protrusion 86 extends into the channel 82 so legs 90 engage and protrude into cutouts 96. Simultaneously, the locking ring 100 snaps over the lips 91 of protrusion 86 to secure and lock the baseplate to the articulation component. When articulation component 72 and baseplate 74 are engaged and locked together, the planar bearing surfaces of both components lie in direct parallel engagement each other. These surfaces are free to slideably engage so the articulation component can rotate relative to the baseplate. One skilled in the art will appreciate that the tolerances of these components could also be modified to make this assembly a non-rotateable assembly. For example, especially tangs 91 can be configured to engage the inner wall of recess 84 and prevent relative movement between the components.

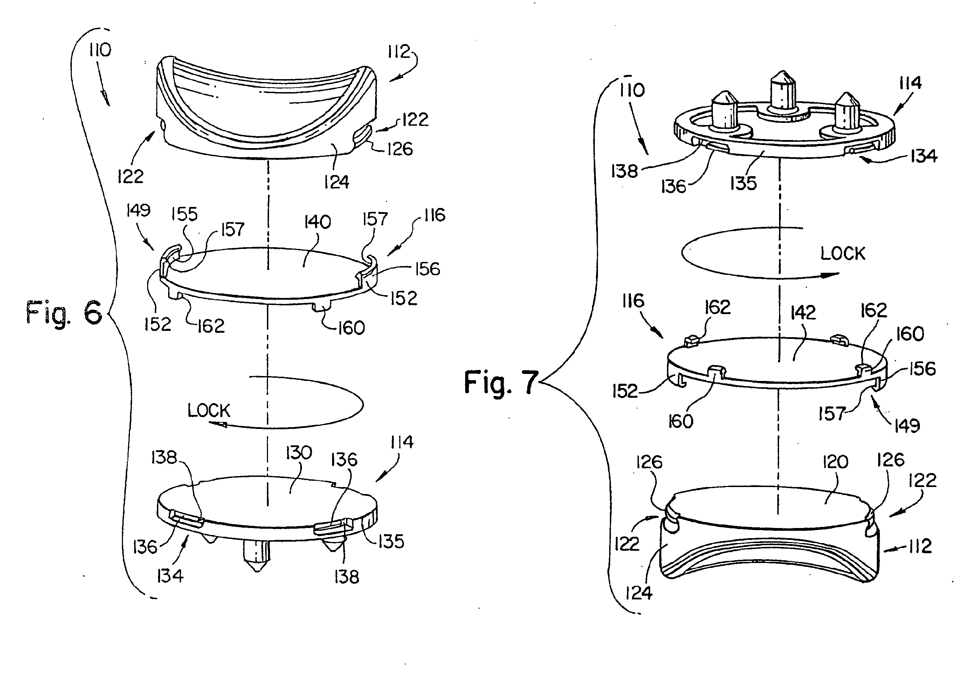

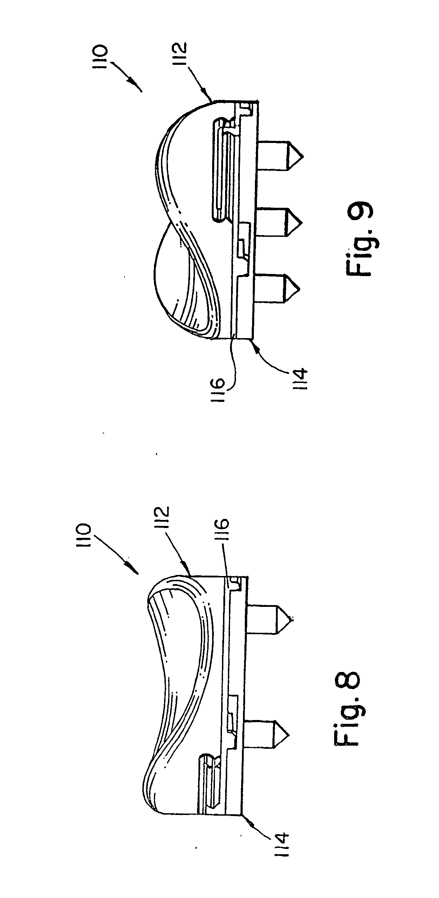

FIGS. 6-9 show another alternate modular knee prosthesis 110 that can be used with the various embodiments of the present invention. The prosthesis includes an articulation component 112, a baseplate 114, and an attachment mechanism 116. The articulation component and baseplate generally have a configuration similar to the articulation component 12 and baseplate 14 shown and described in connection with FIGS. 1-3. The primary differences between these embodiments centers around the attachment mechanism 116 and how it connects the articulation component to the baseplate.

Articulation component 112 has a smooth, planar bearing surface 120. Two oppositely disposed cutouts 122 are formed along the outer perimeter or wall 124 of the articulation component. These cutouts include a ridge or shoulder 126.

Baseplate 114 has a smooth, planar bearing surface 130. Four equally spaced cutouts or recesses 134 are formed along the outer perimeter or wall 135. Each cutout 134 includes a ledge 136 that partially extends around the length of the cutout. A gap or opening 138 is formed between an end wall of the cutout and the end of the ledge 136.

Attachment mechanism 116 enables the articulation component 112 and baseplate 114 to be connectable to and removable from each other. Specifically, the attachment mechanism 116 has a flat, thin disc-shape with a first locking surface or side 140 adapted to engage the bearing surface 120 of the articulation component 112 and a second locking surface or side 142 adapted to engage the bearing surface 130 of the baseplate 114. The attachment mechanism 116 is provided as a completely separate component from both the articulation component 112 and baseplate 114 and has a locking mechanism 149 that enables the attachment mechanism to permanently connect to the articulation component and removeably connect to the baseplate.

The locking mechanism 149 includes two wings or shoulders 152 located on the periphery of the disk. Each wing 152 extends upwardly from the first locking surface 140 and has an elongated, thin, rectangular shape defined by an inside wall 155 and an outside wall 156. A lip or ridge 157 extends along the inside wall 155. The wings extend around the outer perimeter of the first locking surface and thus have a curved shape. The locking mechanism 149 also includes four arms 160 that extend outwardly from the second locking surface 142. These arms have an "L" shape with a lip or tab 162 and are equally spaced around the outer perimeter of the attachment mechanism 116.

In operation, articulation component 112 and baseplate 114 are configured to engage each other in a removable locking or snap-retaining relationship. Specifically, the locking mechanism 149 is shaped and sized to connect to the articulation component 112. As the first locking surface 140 of the locking mechanism 149 is pressed or abutted against the bearing surface 120 of the articulation component 112, wings 152 project into and engage with cutouts 122. As the wings are pressed into the cutouts, the ridges 157 of wings 152 snap over the shoulders 126 to lock the attachment mechanism 116 to the articulation component 112. The wings 152 can be configured to be resilient and slightly deform outwardly so the ridges 157 fit over the shoulders 126.

The connection between the articulation component 112 and the attachment mechanism 116 can be designed to be either permanent (i.e., not removable) or removable. Once the two components are connected, wings 152 are locked into cutouts 122 and prevent the attachment mechanism and articulation component from rotating relative to each other.

As the bearing surface 120 of the articulation component 112 and second locking surface 142 of attachment mechanism 116 are pressed or abutted against the bearing surface 130 of the baseplate 114, the arms 160 on the second locking surface 142 of the attachment mechanism 116 extend through the openings 138 of each cutout 134. The attachment mechanism 116 and attached articulation component 112 are then rotated so the tabs 162 are positioned under ledge 136. FIGS. 6 and 7 illustrate a locking rotation (shown with an arrow and "Lock") needed to connect the articulation component to the baseplate. In this position, the articulation component is engaged and locked with the baseplate. Further, the planar bearing surfaces of both components lie in direct parallel engagement each other. These surfaces slideably engage while the components are locked and unlocked, but otherwise the articulation component does not rotate relative to the baseplate.

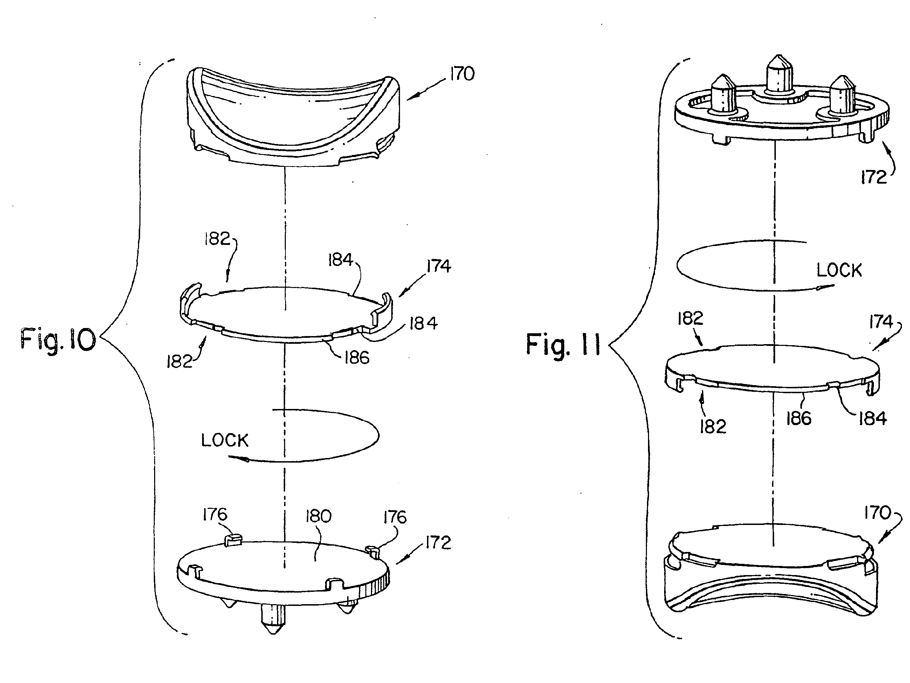

FIGS. 6-9 show an attachment mechanism 116 formed as a disk with a locking mechanism adapted to engage both the articulation component and baseplate. One skilled in the art will appreciate that attachment mechanism can be altered without departing from the scope of the invention. As an example, the components of the locking mechanism can be switched, moved, and altered. FIGS. 10 and 11 show one such embodiment.

In FIGS. 10 and 11, the articulation component 170, baseplate 172, and attachment mechanism 174 generally have a configuration similar to the corresponding components shown and described in connection with FIGS. 6-9. The primary differences between these embodiments centers around the attachment mechanism 174 and how it connects the articulation component to the baseplate. Specifically, the four arms 176 (previously shown in FIGS. 6-9 at 160 on the attachment mechanism 116) now extend outwardly from the bearing surface 180 of the baseplate 172. Further, the cutouts 182 and corresponding openings 184 (previously shown in FIGS. 6-9 at 134 and 138, respectively, on the baseplate 114) are now positioned along an outer perimeter 186 of attachment mechanism 174.

Articulation component 170 engages, locks, unlocks, and disengages from baseplate 172 in a manner similar to the articulation component 112 and baseplate 114 described in FIGS. 6-9. FIGS. 10 and 11 illustrate one example how the attachment mechanism can be altered from another embodiment without departing from the scope of the invention.

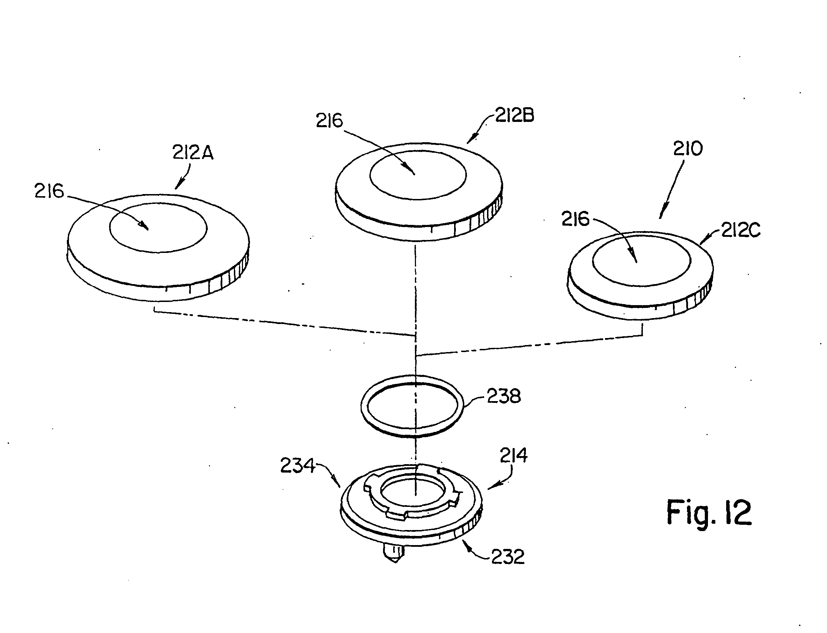

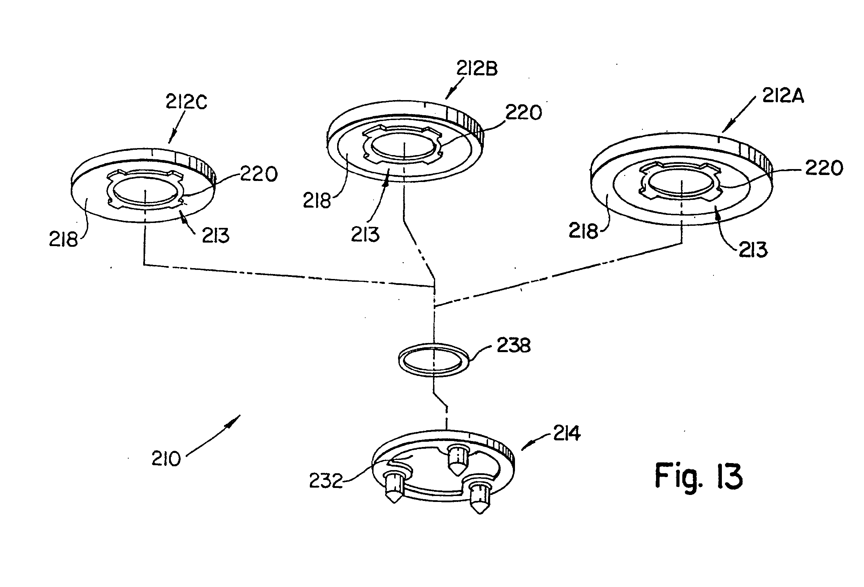

As previously shown and discussed in connection with FIGS 1-3, the modular knee prosthetic system of the present invention can have a plurality of articulation components with different sizes and a plurality of baseplates with different sizes. In these figures, the various articulation components have saddle shapes, while the baseplates use a central pin to engage and connect with the attachment mechanism and accompanying articulation component. One skilled in the art, though, will appreciate that the articulation component, attachment mechanism, and baseplate can be modified without departing from the scope of the invention. FIGS. 12 and 13 illustrate one such example.

FIGS. 12 and 13 show a modular knee prosthetic system or kit 210 having a plurality of individual, implantable patellar prostheses. Prostheses of different sizes are shown wherein each prosthesis includes an articulation or bearing component 212A-212C, an attachment mechanism 213, and a common baseplate 214.

Articulation component 212 includes two primary surfaces: An articulation surface 216 and a planar bearing surface 218 oppositely disposed from the articulation surface. The bearing surface 218 has a configuration similar to the bearing surface 80 of articulation component 72 shown and described in connection with FIGS. 4 and 5. In FIGS. 12 and 13, though, the ring-shaped attachment mechanism 213 (previously shown in FIGS. 4 and 5 at 76) is integrally formed into a circular channel or groove 220 of bearing surface 218.

The articulation surface 216 of the articulation component 212 has a rounded or dome-like shape. Specifically, a tapered or conical section 222 extends inwardly from a side perimeter or wall 224. This tapered section abuts a generally flat top section 226.

Baseplate 214 includes a fixation surface 232 for engaging patellar bone and a planar bearing surface 234. The baseplate 214 has a configuration that is identical to the baseplate 74 shown and described in connection with FIGS. 4 and 5.

As shown in FIGS. 12 and 13, the attachment mechanism 213 includes a circular locking wire or ring 238. This ring can be positioned into and out of the channel 220 of the articulation component to engage and lock the baseplate 214 when the articulation component is connected to the baseplate. The articulation component 212 attaches and detached from the baseplate 214 in a manner similar to the articulation component 72 and baseplate 74 of FIGS. 4 and 5. This ring may also serve as an x-ray marker to aid in the location of the bearing component in the unlikely event that the bearing component should become dislodged from the baseplate component (for example, due to trauma). A metallic ring would be useful if the other components were fabricated from materials such as UHMWPe.

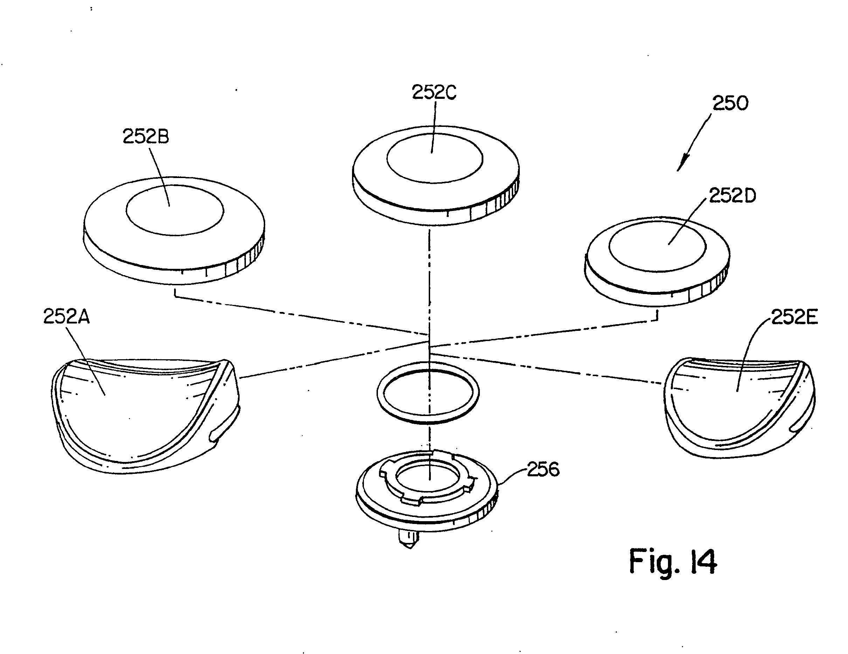

FIG. 14 further illustrates the diversification of the modular knee prosthetic system 250 of the present invention. Prosthetic combinations of different sizes and shapes are available and possible wherein each prosthesis includes an articulation component 252A-252E, an attachment mechanism (not shown), and a baseplate 256. These components attach and detach from one another in a manner similar to the components shown and described in connection with FIGS. 4, 5, 12, and 13.

It is important to note that the prosthetic system 250 of FIG. 14 includes a plurality of articulation components having different sizes and shapes. Components 252A and 252E have articulation surfaces with saddle shapes, whereas components 252B-252D have articulation surfaces with dome-like shapes. One skilled in the art will appreciate that the number and sizes and shapes of both the articulation components and baseplates can increase or decrease to offer a more diversified modular prosthetic knee system.

The articulation component of the present invention can enjoy various degrees of freedom of movement relative to the baseplate. The term "degree of freedom" is used in its ordinary engineering sense to mean freedom of a component to rotate about or translate along a line that is parallel to one axis of a three-axis Cartesian coordinate system fixed in orientation relative to the reference component. The freedom to rotate about such a line comprises one degree of rotational freedom, and the freedom to translate along such a line comprises one translational degree of freedom. A component can enjoy a maximum of six degrees of freedom, in which case the component can rotate about any axis and can translate along any axis. Essentially, a component with six degrees of freedom is unconstrained by any other component.

United States Patent Number 5,702,465 entitled "Patella Prosthesis Having Rotational and Translational Freedom" teaches an articulation component and baseplate having two degrees of freedom. The present invention can be employed with the embodiments taught therein.

Further, the present invention can be utilized with various prosthetic knee designs, including both mobile bearing and fixed knee designs.

Even further, one skilled in the art will appreciate that the attachment mechanism used to connect the articulation component to the baseplate may be modified without departing from the scope of the invention. For example, the male and female components on the articulation component could be switched with the corresponding components on the baseplate.

Further yet, it is important to reiterate that the present invention includes a family of baseplates, a family or articulation components, and a family of attachment mechanisms that all can be produced and packaged separately or together with the intention of producing a modular prosthetic knee system. The articulation components and baseplates can be assembled intra-operatively in a mix and match fashion to meet the needs of the patient. Further, the present invention contemplates multiple components in a family of articulation components and baseplates that can be removed or replaced with like or different components from the family. A large family of components can serve a wide array of patient needs and give the surgeon modularity between components even during intra-operative assembly.

Although illustrative embodiments have been shown and described, a wide range of modifications, changes, and substitutions is contemplated in the foregoing disclosure and in some instances, some features of the embodiments may be employed without a corresponding use of other features. Accordingly, it is appropriate that the appended claims be construed broadly and in a manner consistent with the scope of the embodiments disclosed herein.

## Claims

1. A modular patellar prosthetic system (10, 210, 250), comprising:
at least one baseplate (14, 74, 114, 172, 214, 256) having a fixation surface (32, 232) adapted to engage natural patellar bone (17) and a bearing surface (34, 88, 130, 180, 234) oppositely disposed from the fixation surface (32, 232); **characterised in that** it further comprises a plurality of articulation components (12, 72, 112, 170, 212, 252), each articulation component (12, 72, 112, 170, 212, 252) having a smooth articulation surface (16, 216) adapted to articulate with a femoral component at a patello-femoral joint; and
at least one attachment mechanism (13, 76, 116, 174, 213) separate from the baseplate (14, 74, 114, 172, 214, 256) and articulation component (12, 72, 112, 170, 212, 252), wherein the attachment mechanism (13, 76, 116, 174, 213) connects one of the articulation components (12, 72, 112, 170, 212, 252) to one of the baseplates (14, 74, 114, 172, 214, 256) to form a modular knee prosthesis (70, 110) such that the articulation component (12, 72, 112, 170, 212, 252) can be attached and detached to the baseplate (14, 74, 114, 172, 214, 256).

2. The modular patellar prosthetic system (10, 210, 250) of Claim 1, **characterised in that** the attachment mechanism (13, 76, 116, 174, 213) has a first surface that connects to the articulation component (12, 72, 112, 170, 212, 252) and a second surface that connects to the baseplate (14, 74, 114, 172, 214, 256).

3. The modular patellar prosthetic system (10, 210, 250) of Claim 2, **characterised in that** the first surface permanently connects to the articulation component (12, 72, 112, 170, 212, 252), and the second surface removeably connects to the baseplate (14, 74, 114, 172, 214, 256).

4. The modular patellar prosthetic system (10, 210, 250) of Claim 3, **characterised in that** the attachment mechanism (13, 76, 116, 174, 213) has a disc-shaped body.

5. The modular patellar prosthetic system (10, 210, 250) of Claim 4, **characterised in that** the first surface includes at least one wing (52, 152) adapted to engage the articulation component (12, 72, 112, 170, 212, 252), and the second surface includes a recess (60) adapted to engage and lock with the baseplate (14, 74, 114, 172, 214, 256).

6. The modular patellar prosthetic system (10, 210, 250) of Claim 1, **characterised in that** the articulation components (12, 72, 112, 170, 212, 252) are adapted to be connected to and removed from the baseplate (14, 74, 114, 172, 214, 256) while the baseplate (14, 74, 114, 172, 214, 256) is permanently affixed to natural patellar bone (17).

7. The modular patellar prosthetic system (10, 210, 250) of Claim 6, **characterised in that** the attachment mechanism (13, 76, 116, 174, 213) has a first surface that connects to the articulation component (12, 72, 112, 170, 212, 252), and a second surface that connects to the bearing surface (34, 88, 130, 180, 234) of the baseplate (14, 74, 114, 172, 214, 256).

8. The modular patellar prosthetic system (10, 210, 250) of Claim 7, **characterised in that** the attachment mechanism (13, 76, 116, 174, 213) and baseplate (14, 74, 114, 172, 214, 256) snap-fit and lock together and create a removeable connection.

9. The modular patellar prosthetic system (10, 210, 250) of Claim 1, **characterised in that** the system comprises :
a plurality of baseplates (14, 74, 114, 172, 214, 256) wherein at least two baseplates (14, 74, 114, 172, 214, 256) are provided with different sizes; and
the plurality of articulation components (12, 72, 112, 170, 212, 252), each have a bearing surface (18, 80, 120, 218) adapted to engage the bearing surface (34, 88, 130, 180, 234) of a baseplate (14, 74, 114, 172, 214, 256), wherein at least two articulation components (12, 72, 112, 170, 212, 252) are provided with different sizes.

10. The modular patellar prosthetic system (10, 210, 250) of Claim 9, **characterised in that** the articulation components (12, 72, 112, 170, 212, 252) removeably snap-fit to the baseplates (14, 74, 114, 172, 214, 256).

11. The modular patellar prosthetic system (10, 210, 250) of Clam 9, **characterised in that** the plurality of articulation components (12, 72, 112, 170, 212, 252) includes two articulation components (12, 72, 112, 170, 212, 252) with a saddle shape and two articulation components (12, 72, 112, 170, 212, 252) with a dome-shape.

12. The modular patellar prosthetic system (10, 210, 250) of Claim 11, **characterised in that** each articulation component (12, 72, 112, 170, 212, 252) has a different size and each baseplate (14, 74, 114, 172, 214, 256) has a different size.

13. The modular patellar prosthetic system (10, 210, 250) of Claim 1, **characterised in that** the system further comprises:
a plurality of baseplates (14, 74, 114, 172, 214, 256) wherein at least two baseplates (14, 74, 114, 172, 214, 256) have different sizes;
the plurality of articulation components (12, 72, 112, 170, 212, 252) each have a bearing surface (18, 80, 120, 218) adapted to engage the bearing surface (34, 88, 130, 180, 234) of the baseplate (14, 74, 114, 172, 214, 256), wherein at least two articulation components (12, 72, 112, 170, 212, 252) have different sizes; and
a plurality of attachment mechanisms (13, 76, 116, 174, 213).

14. The modular patellar prosthetic system (10, 210, 250) of Claim 13, **characterised in that** the articulation surfaces (16, 216) of the articulation components (12, 72, 112, 170, 212, 252) include at least two different shapes.

15. The modular patellar prosthetic system (10, 210, 250) of Claim 14, **characterised in that** at least one articulation surface (16, 216) has a saddle shape and at least another articulation surface (16, 216) has a dome-shape.

16. The modular patellar prosthetic system (10, 210, 250) of Claim 15, **characterised in that** each saddle shaped articulation component (12, 72, 112, 170, 212, 252) and each dome-shaped articulation component (12, 72, 112. 170, 212, 252) can attach and detatch from each baseplate (14, 74, 114, 172, 214, 256).

17. The modular patellar prosthetic system (10, 210, 250) of Claim 13, **characterised in that** the articulation component (12, 72, 112, 170, 212, 252), the baseplate (14, 74, 114, 172, 214, 256) and the attachment mechanism (13, 76, 116, 174, 213) are each formed as a separate, unitary member.

18. The modular patellar prosthetic system (10, 210, 250) of Claim 13, **characterised in that** one of the articulation components (12, 72, 112, 170, 212, 252), one of the baseplates (14, 74, 114, 172, 214, 256), and one of the attachment mechanisms (13, 76, 116, 174, 213) connect together to form a single prosthetic patellar implant formed from three separate and different pieces.

19. The modular patellar prosthetic system (10, 210, 250) of Claim 13, **characterised in that** the articulation component (12, 72, 112, 170, 212, 252) slideably rotates relative to the baseplate (14, 74, 114, 172, 214, 256) while the articulation component (12, 72, 112, 170, 212, 252) is connected to the baseplate (14, 74, 114, 172, 214, 256) and the baseplate (14, 74, 114, 172, 214, 256) is permanently affixed to the patella.

## Patentansprüche

1. Modulares Patellaprothesesystem (10, 210, 250), das aufweist:
mindestens eine Grundplatte (14, 74, 114, 172, 214, 256), die eine Befestigungsfläche (32, 232), die eingerichtet ist, mit einem natürlichen Patellaknochen (17) in Eingriff zu treten, und eine Lagerfläche (34, 88, 130, 180, 234) aufweist, die von der Befestigungsfläche (32, 232) gegenüberliegend angeordnet ist; **dadurch gekennzeichnet, daß** es ferner aufweist: mehrere Gelenkkomponenten (12, 72, 112, 170, 212, 252), wobei jede Gelenkkomponente (12, 72, 112, 170, 212, 252) eine glatte Gelenkfläche (16, 216) aufweist, die eingerichtet ist, an einem patellofemoralen Gelenk mit einer femoralen Komponente ein Gelenk zu bilden; und
mindestens einen Verbindungsmechanismus (13, 76, 116, 174, 213), der von der Grundplatte (14, 74, 114, 172, 214, 256) und der Gelenkkomponente (12, 72, 112, 170, 212, 252) getrennt ist, wobei der Verbindungsmechanismus (13, 76, 116, 174, 213) eine der Gelenkkomponenten (12, 72, 112, 170, 212, 252) mit einer der Grundplatten (14, 74, 114, 172, 214, 256) verbindet, um eine modulare Knieprothese (70, 110) zu bilden, so daß die Gelenkkomponente (12, 72, 112, 170, 212, 252) an der Grundplatte (14, 74, 114, 172, 214, 256) angebracht und von ihr gelöst werden kann.

2. Modulares Patellaprothesesystem (10, 210, 250) nach Anspruch 1, **dadurch gekennzeichnet, daß** der Verbindungsmechanismus (13, 76, 116, 174, 213) eine erste Fläche, die mit der Gelenkkomponente (12, 72, 112, 170, 212, 252) verbunden ist, und eine zweite Fläche aufweist, die mit der Grundplatte (14, 74, 114, 172, 214, 256) verbunden ist.

3. Modulares Patellaprothesesystem (10, 210, 250) nach Anspruch 2, **dadurch gekennzeichnet, daß** die erste Fläche dauerhaft mit der Gelenkkomponente (12, 72, 112, 170, 212, 252) verbunden ist, und die zweite Fläche lösbar mit der Grundplatte (14, 74, 114, 172, 214, 256) verbunden ist.

4. Modulares Patellaprothesesystem (10, 210, 250) nach Anspruch 3, **dadurch gekennzeichnet, daß** der Verbindungsmechanismus (13, 76, 116, 174, 213) einen scheibenförmigen Körper aufweist.

5. Modulares Patellaprothesesystem (10, 210, 250) nach Anspruch 4, **dadurch gekennzeichnet, daß** die erste Fläche mindestens einen Flügel (52, 152) aufweist, der eingerichtet ist, mit der Gelenkkomponente (12, 72, 112, 170, 212, 252) in Eingriff zu treten, und die zweite Fläche eine Aussparung (60) aufweist, die eingerichtet ist, mit der Grundplatte (14, 74, 114, 172, 214, 256) in Eingriff zu treten und einzurasten.

6. Modulares Patellaprothesesystem (10, 210, 250) nach Anspruch 1, **dadurch gekennzeichnet, daß** die Gelenkkomponenten (12, 72, 112, 170, 212, 252) eingerichtet sind, mit der Grundplatte (14, 74, 114, 172, 214, 256) verbunden und von ihr gelöst zu werden, während die Grundplatte (14, 74, 114, 172, 214, 256) dauerhaft am natürlichen Patellaknochen (17) befestigt ist.

7. Modulares Patellaprothesesystem (10, 210, 250) nach Anspruch 6, **dadurch gekennzeichnet, daß** der Verbindungsmechanismus (13, 76, 116, 174, 213) eine erste Fläche, die mit der Gelenkkomponente (12, 72, 112, 170, 212, 252) verbunden ist, und eine zweite Fläche aufweist, die mit der Lagerfläche (34, 88, 130, 180, 234) der Grundplatte (14, 74, 114, 172, 214, 256) verbunden ist.

8. Modulares Patellaprothesesystem (10, 210, 250) nach Anspruch 7, **dadurch gekennzeichnet, daß** der Verbindungsmechanismus (13, 76, 116, 174, 213) und die Grundplatte (14, 74, 114, 172, 214, 256) ineinander einschnappen und einrasten und eine lösbare Verbindung bilden.

9. Modulares Patellaprothesesystem (10, 210, 250) nach Anspruch 1, **dadurch gekennzeichnet, daß** das System aufweist:
mehrere Grundplatten (14, 74, 114, 172, 214, 256), wobei mindestens zwei Grundplatten (14, 74, 114, 172, 214, 256) mit unterschiedlichen Größen vorgesehen sind; und
die mehreren Gelenkkomponenten (12, 72, 112, 170, 212, 252) jeweils eine Lagerfläche (18, 80, 120, 218) aufweisen, die eingerichtet ist, mit der Lagerfläche (34, 88, 130, 180, 234) einer Grundplatte (14, 74, 114, 172, 214, 256) in Eingriff zu treten, wobei mindestens zwei Gelenkkomponenten (12, 72, 112, 170, 212, 252) mit unterschiedlichen Größen vorgesehen sind.

10. Modulares Patellaprothesesystem (10, 210, 250) nach Anspruch 9, **dadurch gekennzeichnet, daß** die Gelenkkomponenten (12, 72, 112, 170, 212, 252) lösbar an den Grundplatten (14, 74, 114, 172, 214, 256) einschnappen.

11. Modulares Patellaprothesesystem (10, 210, 250) nach Anspruch 9, **dadurch gekennzeichnet, daß** die mehreren Gelenkkomponenten (12, 72, 112, 170, 212, 252) zwei Gelenkkomponenten (12, 72, 112, 170, 212, 252) mit einer Sattelform und zwei Gelenkkomponenten (12, 72, 112, 170, 212, 252) mit einer Kuppelform aufweisen.

12. Modulares Patellaprothesesystem (10, 210, 250) nach Anspruch 11, **dadurch gekennzeichnet, daß** jede Gelenkkomponente (12, 72, 112, 170, 212, 252) eine unterschiedliche Größe aufweist und jede Grundplatte (14, 74, 114, 172, 214, 256) eine unterschiedliche Größe aufweist.

13. Modulares Patellaprothesesystem (10, 210, 250) nach Anspruch 1, **dadurch gekennzeichnet, daß** das System ferner aufweist:
mehrere Grundplatten (14, 74, 114, 172, 214, 256), wobei mindestens zwei Grundplatten (14, 74, 114, 172, 214, 256) unterschiedlichen Größen aufweisen;
wobei die mehreren Gelenkkomponenten (12, 72, 112, 170, 212, 252) jeweils eine Lagerfläche (18, 80, 120, 218) aufweisen, die eingerichtet ist, mit der Lagerfläche (34, 88, 130, 180, 234) der Grundplatte (14, 74, 114, 172, 214, 256) in Eingriff zu treten, wobei mindestens zwei Gelenkkomponenten (12, 72, 112, 170, 212, 252) unterschiedliche Größen aufweisen; und
mehrere Verbindungsmechanismen (13, 76, 116, 174, 213).

14. Modulares Patellaprothesesystem (10, 210, 250) nach Anspruch 13, **dadurch gekennzeichnet, daß** die Gelenkflächen (16, 216) der Gelenkkomponenten (12, 72, 112, 170, 212, 252) mindestens zwei unterschiedliche Formen aufweisen.

15. Modulares Patellaprothesesystem (10, 210, 250) nach Anspruch 14, **dadurch gekennzeichnet, daß** mindestens eine Gelenkfläche (16, 216) eine Sattelform aufweist und mindestens eine andere Gelenkfläche (16, 216) eine Kuppelform aufweist.

16. Modulares Patellaprothesesystem (10, 210, 250) nach Anspruch 15, **dadurch gekennzeichnet, daß** jede sattelförmige Gelenkkomponente (12, 72, 112, 170, 212, 252) und jede kuppelförmige Gelenkkomponente (12, 72, 112, 170, 212, 252) an jeder Grundplatte (14, 74, 114, 172, 214, 256) angebracht und von ihr gelöst werden kann.

17. Modulares Patellaprothesesystem (10, 210, 250) nach Anspruch 13, **dadurch gekennzeichnet, daß** die Gelenkkomponente (12, 72, 112, 170, 212, 252), die Grundplatte (14, 74, 114, 172, 214, 256) und der Verbindungsmechanismus (13, 76, 116, 174, 213) jeweils als ein separates, einheitliches Element ausgebildet sind.

18. Modulares Patellaprothesesystem (10, 210, 250) nach Anspruch 13, **dadurch gekennzeichnet, daß** eine der Gelenkkomponenten (12, 72, 112, 170, 212, 252), eine der Grundplatten (14, 74, 114, 172, 214, 256), und einer der Verbindungsmechanismen (13, 76, 116, 174, 213) miteinander verbunden sind, um ein einzelnes prothetisches Patellaimplantat zu bilden, das von drei separaten und unterschiedlichen Stücken gebildet wird.

19. Modulares Patellaprothesesystem (10, 210, 250) nach Anspruch 13, **dadurch gekennzeichnet, daß** die Gelenkkomponente (12, 72, 112, 170, 212, 252) relativ zur Grundplatte (14, 74, 114, 172, 214, 256) gleitend rotiert, während die Gelenkkomponente (12, 72, 112, 170, 212, 252) mit der Grundplatte (14, 74, 114, 172, 214, 256) verbunden ist und die Grundplatte (14, 74, 114, 172, 214, 256) dauerhaft an der Patella befestigt ist.

## Revendications

1. Système prothétique patellaire modulaire (10, 210, 250) comprenant:
au moins une plaque de base (14, 74, 114, 172, 214, 256) ayant une surface de fixation (32, 232) adaptée pour engager un os patellaire naturel (17) et une surface d'appui (34, 88, 130, 180, 234) disposée de manière opposée à la surface de fixation (32, 232); **caractérisé en ce qu'**il comprend en plus une pluralité de composants d'articulation (12, 72, 112, 170, 212, 252), chaque composant d'articulation (12, 72, 112, 170, 212, 252) ayant une surface d'articulation lisse (16, 216) adaptée pour s'articuler avec un composant fémoral au niveau d'un joint fémoro-rotulien; et
au moins un mécanisme de raccordement (13, 76, 116, 174, 213) séparé de la plaque de base (14, 74, 114, 172, 214, 256) et du composant d'articulation (12, 72, 112, 170, 212, 252), où le mécanisme de raccordement (13, 76, 116, 174, 213) relie l'un des composants d'articulation (12, 72, 112, 170, 212, 252) à l'une des plaques de base (14, 74, 114, 172, 214, 256) pour former une prothèse modulaire de genou (70, 110) de telle sorte que le composant d'articulation (12, 72, 112, 170, 212, 252) puisse être relié à et détaché de la plaque de base (14, 74, 114, 172, 214, 256).

2. Système prothétique patellaire modulaire (10, 210, 250) de la revendication 1, **caractérisé en ce que** le mécanisme de raccordement (13, 76, 116, 174, 213) a une première surface qui se relie au composant d'articulation (12; 72, 112, 170, 212, 252) et une deuxième surface qui se relie à la plaque de base (14, 74, 114, 172, 214, 256).

3. Système prothétique patellaire modulaire (10, 210, 250) de la revendication 2, **caractérisé en ce que** la première surface se relie de manière permanente au composant d'articulation (12, 72, 112, 170, 212, 252), et la deuxième surface se relie de manière amovible à la plaque de base (14, 74, 114, 172, 214, 256).

4. Système prothétique patellaire modulaire (10, 210, 250) de la revendication 3, **caractérisé en ce que** le mécanisme de raccordement (13, 76, 116, 174, 213) a un corps en forme de disque.

5. Système prothétique patellaire modulaire (10, 210, 250) de la revendication 4, **caractérisé en ce que** la première surface inclut au moins une aile (52, 152) adaptée pour être en prise avec le composant d'articulation (12, 72, 112, 170, 212, 252), et la deuxième surface inclut un évidement (60) adapté pour s'engager et se verrouiller avec la plaque de base (14, 74, 114, 172, 214, 256).

6. Système prothétique patellaire modulaire (10, 210, 250) de la revendication 1, **caractérisé en ce que** les composants d'articulation (12, 72, 112, 170, 212, 252) sont adaptés pour être reliés à et retirés de la plaque de base (14, 74, 114, 172, 214, 256) tandis que la plaque de base (14, 74, 114, 172, 214, 256) est fixée de manière permanente à un os patellaire naturel (17).

7. Système prothétique patellaire modulaire (10, 210, 250) de la revendication 6, **caractérisé en ce que** le mécanisme de raccordement (13, 76, 116, 174, 213) a une première surface qui se relie au composant d'articulation (12, 72, 112, 170, 212, 252), et une deuxième surface qui se relie à la surface d'appui (34, 88, 130, 180, 234) de la plaque de base (14, 74, 114, 172, 214, 256).

8. Système prothétique patellaire modulaire (10, 210, 250) de la revendication 7, **caractérisé en ce que** le mécanisme de raccordement (13, 76, 116, 174, 213) et la plaque de base (14, 74, 114, 172, 214, 256) sont emboîtés par pression et sont verrouillés ensemble et créent une connexion amovible.

9. Système prothétique patellaire modulaire (10, 210, 250) de la revendication 1, **caractérisé en ce que** le système comprend:
une pluralité de plaques de base (14, 74, 114, 172, 214, 256) où au moins deux plaques de base (14, 74, 114, 172, 214, 256) ont des tailles différentes; et
la pluralité de composants d'articulation (12, 72, 112, 170, 212, 252), chacun a une surface d'appui (18, 80, 120, 218) adaptée pour être en prise avec la surface d'appui (34, 88, 130, 180, 234) d'une plaque de base (14, 74, 114, 172, 214, 256), où au moins deux composants d'articulation (12, 72, 112, 170, 212, 252) ont des tailles différentes.

10. Système prothétique patellaire modulaire (10, 210, 250) de la revendication 9, **caractérisé en ce que** les composants d'articulation (12, 72, 112, 170, 212, 252) s'emboîtent par pression de manière amovible aux plaques de base (14, 74, 114, 172, 214, 256).

11. Système prothétique patellaire modulaire (10, 210, 250) de la revendication 9, **caractérisé en ce que** la pluralité de composants d'articulation (12, 72, 112, 170, 212, 252) inclut deux composants d'articulation (12, 72, 112, 170, 212, 252) avec une forme de selle et deux composants d'articulation (12, 72, 112, 170, 212, 252) avec une forme de dôme.

12. Système prothétique patellaire modulaire (10, 210, 250) de la revendication 11, **caractérisé en ce que** chaque composant d'articulation (12, 72, 112, 170, 212, 252) a une taille différente et chaque plaque de base (14, 74, 114, 172, 214, 256) a une taille différente.

13. Système prothétique patellaire modulaire (10, 210, 250) de la revendication 1, **caractérisé en ce que** le système comprend en plus:
une pluralité de plaques de base (14, 74, 114, 172, 214, 256) où au moins deux plaques de base (14, 74, 114, 172, 214, 256) ont des tailles différentes;
la pluralité de composants d'articulation (12, 72, 112, 170, 212, 252) chacun a une surface d'appui (18, 80, 120, 218) adaptée pour être en prise avec la surface d'appui (34, 88, 130, 180, 234) de la plaque de base (14, 74, 114, 172, 214, 256), où au moins deux composants d'articulation (12, 72, 112, 170, 212, 252) ont des tailles différentes; et
une pluralité de mécanismes de raccordement (13, 76, 116, 174, 213).

14. Système prothétique patellaire modulaire (10, 210, 250) de la revendication 13, **caractérisé en ce que** les surfaces d'articulation (16, 216) des composants d'articulation (12, 72, 112, 170, 212, 252) incluent au moins deux formes différentes.

15. Système prothétique patellaire modulaire (10, 210, 250) de la revendication 14, **caractérisé en ce qu'**au moins une surface d'articulation (16, 216) a une forme de selle et au moins une autre surface d'articulation (16, 216) a une forme de dôme.

16. Système prothétique patellaire modulaire (10, 210, 250) de la revendication 15, **caractérisé en ce que** chaque composant d'articulation en forme de selle (12, 72, 112, 170, 212, 252) et chaque composant d'articulation en forme de dôme (12, 72, 112, 170, 212, 252) peut être relié et détaché de chaque plaque de base (14, 74, 114, 172, 214, 256).

17. Système prothétique patellaire modulaire (10, 210, 250) de la revendication 13, **caractérisé en ce que** le composant d'articulation (12, 72, 112, 170, 212, 252), la plaque de base (14, 74, 114, 172, 214, 256) et le mécanisme de raccordement (13, 76, 116, 174, 213) sont formés chacun comme un élément unitaire, séparé.

18. Système prothétique patellaire modulaire (10, 210, 250) de la revendication 13, **caractérisé en ce que** l'un des composants d'articulation (12, 72, 112, 170, 212, 252), l'une des plaques de base (14, 74, 114, 172, 214, 256), et l'un des mécanismes de raccordement (13, 76, 116, 174, 213) se relient entre eux pour former un implant patellaire prothétique unique formé à partir de trois pièces différentes et séparées.

19. Système prothétique patellaire modulaire (10, 210, 250) de la revendication 13, **caractérisé en ce que** le composant d'articulation (12, 72, 112, 170, 212, 252) tourne de manière coulissante par rapport à la plaque de base (14, 74, 114, 172, 214, 256) tandis que le composant d'articulation (12, 72, 112, 170, 212, 252) est relié à la plaque de base (14, 74, 114, 172, 214, 256) et la plaque de base (14, 74, 114, 172, 214, 256) est fixée de manière permanente à la rotule.
